# EUROPÄISCHE PATENTSCHRIFT

(11) **EP 3 141 267 B1**
(45) Veröffentlichungstag und Bekanntmachung des Hinweises auf die Patenterteilung: **20.11.2019**
(21) Anmeldenummer: 16187651.1
(22) Anmeldetag: 07.09.2016
(51) Int. Cl.: A61L 24/04

(54) **POLYMETHYLMETHACRYLAT-KNOCHENZEMENT MIT EINSTELLBARER INITIALER VISKOSITÄT UND EIN VERFAHREN ZUR HERSTELLUNG EINES KNOCHENZEMENTTEIGS MIT VARIABLER INITIALER VISKOSITÄT**
POLYMETHYLMETHACRYLATE BONE CEMENT WITH ADJUSTABLE INITIAL VISCOSITY AND A METHOD FOR PRODUCING A BONE CEMENT PASTE WITH VARIABLE INITIAL VISCOSITY
CIMENT OSSEUX EN POLYMÉTHACRYLATE DE MÉTHYLE AYANT UNE VISCOSITÉ INITIALE MODIFIABLE ET PROCÉDÉ DE PRODUCTION D'UNE PÂTE DE CIMENT OSSEUX AYANT UNE VISCOSITÉ INITIALE VARIABLE

(30) Priorität: 10.09.2015 DE 102015217315
(43) Veröffentlichungstag der Anmeldung: 15.03.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Heraeus IP

(56) Entgegenhaltungen:
- EP-A1- 3 093 067
- EP-A2- 2 052 747
- EP-A2- 2 055 324
- EP-A2- 2 269 718
- US-A1- 2004 157 954
- US-A1- 2010 228 358

## Beschreibung

Gegenstand der Erfindung ist ein Kit zur Herstellung von polymerisierbaren Knochenzementen, umfassend die Komponenten A und die Komponente B, wobei die Komponente A als Pulver vorliegt und umfasst
(a.1) mindestens ein pulverförmiges Polymethylmethacrylat-Copolymer, wobei das pulverförmige Polymethylmethacrylat-Copolymer mindestens ein partikuläres Polymethylmethacrylat-Copolymer mit einer Molmasse Mn größer gleich 200.000 g/mol bis 1.000.000 g/mol umfasst, und das Polymethylmethacrylat-Copolymer erhältlich ist durch Polymerisation eines Gemisches von größer gleich 90,0 Gew.-% Methylmethacrylat und kleiner gleich 10,0 Gew.-% bis größer gleich 1 Gew.-% eines oder mehrerer Comonomere, und die Gesamtzusammensetzung in Bezug auf dieses Gemisch 100 Gew.-% beträgt,
(a.2) mindestens einen pulverförmigen Röntgenopaker, und
(a.3) mindestens einen Polymerisationsinitiator; und
Komponente B als Flüssigkeit vorliegt und umfasst
(b.1) mindestens ein radikalisch polymerisierbares Monomer,
(b.2) optional mindestens einen Polymerisationsbeschleuniger und
(b.3) optional mindestens einen Stabilisator, und
wobei die Komponente A im Innenraum (13) einer Kartusche (1) vorliegt, wobei die Kartusche (1) an ihrem einen Ende einen Kartuschenanschluss mit Innengewinde (8) und an ihrem anderen, gegenüberliegenden Ende einen Austragskolben (2) aufweist, wobei durch eine Durchführung in dem Austragskolben (2) eine Mischstange (4) an eine Mischeinrichtung im Inneren (13) der Kartusche anbringbar ist oder angeordnet ist, wobei die Mischeinrichtung von außen durch Bewegen der Mischstange (4) entlang einer Achse des Innenraumes (13) bedienbar ist, und wobei die Komponente B in einem Einweg oder mehrfach verwendbaren Monomer-Behälter enthalten ist, wobei die Komponente B in einer Glasampulle mit abbrechbarem Ampullenkopf vorliegt, wobei die Glasampulle, die Kartusche (1) und/oder eine Dosiereinrichtung, die zwischen dem Monomer-Behälter und der Kartusche angeordnet ist, Markierungen aufweisen, wobei an den Markierungen die Zugabemenge der Komponente B zur Einstellung des Gewichtsverhältnisses oder das dem Gewichtsverhältnis entsprechenden Volumenverhältnis der Komponente A umfassend das Polymethylmethacrylat-Copolymer zur Komponente B umfassend das radikalisch polymerisierbare Monomer von etwa 2,0 bis 3,4 zu 1,0 in Skalenteilen der Markierung ablesbar ist.

Ebenfalls Gegenstand der Erfindung ist ein Verfahren zur Herstellung eines polymerisierbaren Knochenzementes unter Verwendung des Kits, indem zwei Komponenten A und B miteinander gemischt werden, wobei
Komponente B als Flüssigkeit vorliegt und umfasst
(b.1) mindestens ein radikalisch polymerisierbares Monomer,
(b.2) mindestens einen Polymerisationsbeschleuniger und
(b.3) mindestens einen Stabilisator,
Komponente A als Pulver vorliegt und umfasst
(a.1) mindestens ein in (b.1) lösliches pulverförmiges Polymethylmethacrylat-Copolymer oder ein pulverförmiges Gemisch umfassend in (b.1) lösliche Polymethylmethacrylat-Copolymere, wobei das Polymethylmethacrylat-Copolymer umfasst mindestens ein partikuläres Polymethylmethacrylat-Copolymer mit einer Molmasse größer gleich 200.000 g/mol und das Polymethylmethacrylat-Copolymer erhältlich ist durch Polymerisation eines Gemisches von größer gleich 90,0 Gew.-% Methylmethacrylat und kleiner gleich 10,0 Gew.-% bis größer gleich 1 Gew.-% eines oder mehrerer Comonomere, und die Gesamtzusammensetzung des Polymethylmethacrylat-Copolymers in Bezug auf dieses Gemisch 100 Gew.-% beträgt,
(a.2) mindestens einen pulverförmigen Röntgenopaker, und
(a.3) mindestens einen Polymerisationsinitiator; und
wobei das Gewichtsverhältnis der Komponente A umfassend das Polymethylmethacrylat-Copolymer zur Komponente B umfassend das radikalisch polymerisierbare Monomer von etwa 2,0 bis 3,4 zu 1,0 beträgt und
die Komponenten A und B im Gewichtsverhältnis ausgewählt aus
a) kleiner 2,2 zu 1,0 oder
b) von 2,2 bis kleiner 3,3 zu 1,0, oder
c) von größer gleich 3,3 zu 1,0 gemischt werden.

Beschrieben wird ein polymerisierbarer Polymethylmethacrylat-Knochenzement, bei dem die initiale Viskosität des Zementteigs gesteuert werden kann. Der polymerisierbare Knochenzement entspricht einer Zusammensetzung umfassend ein radikalisch polymerisierbares Monomer, ein in dem Monomer lösliches pulverförmiges Polymethylmethacrylat-Copolymer oder Gemisch umfassend Polymethylmethacrylat-Copolymere, nachfolgend Polymethylmethacrylat-Copolymer genannt, einen Polymerisationsinitiator, einen Röntgenopaker, wobei das pulverförmige Polymethylmethacrylat-Copolymer mindestens ein partikuläres Polymethylmethacrylat-Copolymer mit einer Molmasse größer gleich 200.000 g/mol umfasst, und das Polymethylmethacrylat-Copolymer erhältlich ist durch Polymerisation eines Gemisches von größer gleich 90,0 Gewichtsprozent Methylmethacrylat und kleiner gleich 10,0 Gewichtsprozent eines oder mehrerer Comonomere, wobei das Gewichtsverhältnis der Komponente A umfassend mindestens ein Polymethylmethacrylat-Copolymer, einen Röntgenopaker und einen Polymerisationsinitiator, insbesondere Dibenzoylperoxid, zur Komponente B umfassend ein radikalisch polymerisierbares Monomer, einen Stabilisator und einem Polymerisationsbeschleuniger, insbesondere ein aromatisches Amin, von etwa 2,0 bis 3,4:1,0 beträgt, um die initiale Viskosität des Knochenzementteigs zu steuern, der durch Vermischung der vorgenannten Komponenten A und B gebildet wird.

Beschrieben wird ein Verfahren zur Herstellung des Knochenzementes als auch die Verwendung zur Einstellung der variablen, initialen Viskosität als auch ein Kit zur Verwendung in diesem Verfahren.

Polymethylmethacrylat-(PMMA)-Knochenzemente gehen auf die grundlegenden Arbeiten von J. Charnley zurück (Charnley, J.: Anchorage of the femoral head prothesis of the shaft of the femur. J. Bone Joint Surg. 42 (1960) 28-30.). PMMA-Knochenzemente bestehen aus einer flüssigen Monomerkomponente und einer Pulverkomponente. Die Monomer-komponente enthält im Allgemeinen das Monomer Methylmethacrylat und einen darin gelösten Aktivator. Die Pulverkomponente, auch als Knochenzementpulver bezeichnet, weist ein oder mehrere Polymere, wie PMMA, das auf Basis von Methylmethacrylat hergestellt ist, einen Röntgenopaker und den Initiator auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Die radikalische Polymerisation des Methylmethacrylates wird durch beim Vermischen gebildete Radikale initiiert. Mit fortschreitender Polymerisation des Methylmethacrylates erhöht sich die Viskosität des Zementteigs bis dieser erstarrt.

Ein Einteilung der Knochenzemente erfolgt u.a. nach dem zeitlichen Erreichen der Klebfreiheit des Knochenzementteigs der PMMA-Knochenzemente in hochviskose, mittelviskose und in niedrigviskose Knochenzemente (Kühn, K.-D.: Knochenzemente für die Endoprothetik. Springer Verlag, 2001, 18-19.). Hochviskose Knochenzemente erreichen die Klebfreiheit nach 1,0 bis 1,5 min. Als mittelviskos werden Zemente bezeichnet, bei denen die Klebfreiheit zwischen 1,5 min und 3,0 min eintritt. Niedrigviskos sind Zemente bei denen der Zementteig nach mehr als 3,0 min klebfrei ist. Der Begriff "Klebfreiheit" ist in der ISO5833 definiert als der Zeitpunkt nach der Vermischung des Zementpulvers mit der Monomerflüssigkeit ab dem die Zementteigoberfläche mit der behandschuhten Hand ohne Ankleben des Handschuhes berührt werden kann.

Der Knochenzementteig ist erst ab Erreichen der Klebfreiheit applizierbar. Das bedeutet, der Zeitpunkt der Klebfreiheit zeigt den Beginn der Verarbeitungsphase des Polymethylmethacrylat-Knochenzements an. Hochviskose und mittelviskose Polymethylmethacrylat-Knochenzemente finden vor allem bei der mechanischen Fixierung von großen Gelenkendoprothesen, zum Beispiel von Femur- und Tibia-Komponenten von Kniegelenkendoprothesen, Verwendung. Niedrigviskose Polymethylmethacrylat-Knochenzemente werden bei kleinen Gelenkendoprothesen, zum Beispiel Schultergelenkendoprothesen, eingesetzt.

Die Einstellung der initialen Viskosität erfolgt bei allen bisher bekannten Zementen im Wesentlichen über speziell zusammengesetzte und aufeinander abgestimmte Zementpulver mit einem definierten Gehalt an Dibenzoylperoxid, der sehr genau festgelegt ist.

Die initiale Viskosität des Polymethylmethacrylat-Knochenzementes ist nur mit sehr viel analytischem Aufwand über längere Zeiträume unter technischen Bedingungen reproduzierbar. Bisher ist es bei allen industriell gefertigten Polymethylmethacrylat-Knochenzementen üblich, dass vom Hersteller durch die Zusammensetzung des Zementpulvers die initiale Viskosität des Polymethylmethacrylat-Knochenzementes vorgegeben wird. Der medizinische Anwender hat bisher keine Möglichkeit, die Viskosität des Polymethylmethacrylat-Knochenzementes entsprechend seinen Bedürfnissen während der OP selbst einzustellen. Insoweit sind die bekannten Knochenzemente in ihrer spezifischen Zusammensetzung begrenzt auf ein festes Mischungsverhältnis der Monomere und der pulverförmigen PMMA-Polymerkomponente.

EP2269718 beschreibt einen Kit zum sterilen und blasenfreien Mischen und Austragen von Knochenzement.

Eine zu Grunde liegende Aufgabe ist es eine Zusammensetzung eines Polymethylmethacrylat-Copolymer Knochenzements zu entwickeln, bei dem mit einem einzigen universellen Zementpulver der medizinische Anwender die initiale Viskosität entsprechend seinen Bedürfnissen selbst einstellen kann. Des Weiteren bestand die Aufgabe ein Verfahren zur Anwendung des universell anwendbaren Knochenzementes bereitzustellen als auch ein Kit in dem dieses universelle Zementpulver und die Monomerkomponente vorliegen und in denen sie direkt angewendet werden können.

Die Aufgabe der Erfindung wurde mit einem Kit nach Anspruch 1 sowie einem Verfahren nach Anspruch 2 gelöst. Weitere bevorzugte Ausführungsformen sind in den Unteransprüchen und detailliert in der Beschreibung offenbart.

Kern ist ein spezifisches, universell einsetzbares pulverförmiges Polymethylmethacrylat-Copolymer, insbesondere als eine Komponente A mit einem spezifischen Gehalt an Polymerisationsinitiator und einem Röntgenopaker sowie optional einer pharmazeutisch wirksamen Substanz, das zusammen mit einem radikalisch polymerisierbaren Monomer zur Herstellung von Knochenzementen mit einer initialen Viskosität von niedrigviskos, über mittelviskos bis hochviskos eingestellt werden kann.

Die Ausführung basiert auf dem überraschenden Befund, dass mit dem zusammengesetzten Knochenzementpulver durch Variation des Gewichtsverhältnisses von Zementpulver (Komponente A) zu Monomerflüssigkeit (Komponente B) die initiale Viskosität des aus dem Zementpulver und der Monomerflüssigkeit gebildeten Zementteigs gezielt eingestellt werden kann. Es sind dadurch keine für die jeweilige Viskosität speziell zusammengesetzte Zementpulver notwendig. Daher erspart die Zusammensetzung dem Anwender den Kauf diverser zur Einstellung einer spezifischen Viskosität bislang notwendigen Produkte, weil er nun mit einem Produkt die gesamte Bandbreite an gewünschter Viskosität des Knochenzementes unmittelbar bei der Verarbeitung einstellen kann.

Beschrieben wird eine Zusammensetzung zur Verwendung als polymerisierbarer Knochenzement, insbesondere ein polymerisierbarer Knochenzement, umfassend
(i) mindestens ein radikalisch polymerisierbares Monomer, insbesondere mindestens Methylmethacrylat,
(ii) mindestens ein in (i) lösliches pulverförmiges Polymethylmethacrylat-Copolymer oder Gemische umfassend Polymethylmethacrylat-Copolymere,
(iii) mindestens einen Polymerisationsinitiator, insbesondere von 0,6 bis 2,5 Gew.-%, vorzugsweise von 0,8 bis 2,5 Gew.-%, bevorzugt von 0,8 bis 2,0 Gew.-% in Bezug auf die Gesamtzusammensetzung von 100 Gew.-%, vorzugsweise von 0,6 bis 0,8 bis 1,4 bis 1,95 Gew.-% Dibenzoylperoxid in Bezug auf die Gesamtzusammensetzung, besonders bevorzugt von 0,65 bis 1,92 Gew.-% in Bezug auf die Gesamtzusammensetzung,
(iv) mindestens einen Röntgenopaker, wobei
das (ii) pulverförmige Polymethylmethacrylat-Copolymer mindestens ein partikuläres Polymethylmethacrylat-Copolymer mit einer Molmasse Mn größer gleich 200.000 g/mol bis 1.000.000 g/mol, insbesondere bis 500.000 g/mol umfasst, und das Polymethylmethacrylat-Copolymer erhältlich ist durch Polymerisation eines Gemisches von größer gleich 90,0 Gewichtsprozent Methylmethacrylat und kleiner gleich 10,0 Gewichtsprozent eines oder mehrerer Comonomere, die insbesondere nicht Methylmethacrylat entsprechen, und die Gesamtzusammensetzung des Polymethylmethacrylat-Copolymers oder von Gemischen enthaltend mindestens ein Copolymer in Bezug auf dieses Gemisch 100 Gew.-% beträgt, wobei die Zusammensetzung erhältlich ist, indem zwei Komponenten A und B gemischt werden,
wobei Komponente A als Pulver vorliegt und umfasst
(a.1) mindestens ein pulverförmiges Polymethylmethacrylat-Copolymer oder Gemische umfassend mindestens ein Polymethylmethacrylat-Copolymer,
(a.2) mindestens einen pulverförmigen Röntgenopaker, und
(a.3) mindestens einen Polymerisationsinitiator; insbesondere Dibenzoylperoxid, und Komponente B als Flüssigkeit vorliegt und umfasst
(b.1) mindestens ein radikalisch polymerisierbares Monomer,
(b.2) optional mindestens einen Polymerisationsbeschleuniger, insbesondere aromatisches Amin, bevorzugt N,N-Dimethyl-p-toluidin, und
(b.3) optional mindestens einen Stabilisator,
wobei das Gewichtsverhältnis der Komponente A umfassend das pulverförmige Polymethylacrylat-copolymer zur Komponente B umfassend das radikalisch polymerisierbare Monomer von etwa 2,0 bis 3,4 zu 1,0 beträgt, wobei das Gewichtsverhältnis der Komponente A zur Komponente B ausgewählt ist aus
a) kleiner 2,2 zu 1,0, oder
b) von 2,2 bis kleiner 3,3 zu 1,0, oder
c) von größer gleich 3,3 zu 1,0.

Dabei ist es besonders bevorzugt, wenn die Komponenten A und B im Gewichtsverhältnis von a) kleiner 2,2 zu 1,0, insbesondere von 2,0 bis kleiner 2,2 zu 1,0, oder b) von 2,2 bis kleiner 3,3 zu 1,0, insbesondere von größer 2,2 bis 3,2 zu 1,0 oder c) von größer gleich 3,3 zu 1,0 vorliegen, insbesondere von 3,3 bis kleiner gleich 3,4 zu 1,0 vorliegen, wobei insbesondere die Komponente A von 1,0 bis 2,5 Gew.-% mindestens einen Polymerisationsinitiator insbesondere Dibenzoylperoxid in Bezug auf die Gesamtzusammensetzung der Komponente A von 100 Gew.-% aufweist.

Bevorzugte Comonomere umfassen Styren, Ethylacrylat, Methylacrylat oder Mischungen enthaltend mindestens zwei der Comonomere. Die Herstellung der pulverförmigen Polymethylmethacrylat-Copolymere erfolgt vorzugsweise in einer Emulsionspolymerisation. Besonders bevorzugt wird mindestens ein Comonomer ausgewählt aus mindestens einem Alkylacrylat mit 1 bis 5 C-Atomen in der Alkyl-Gruppe, insbesondere Methylacrylat, Ethylmethacylat, Phenylalklylen mit 8 bis 20 C-Atomen, insbesondere Phenylethen (Styren), Dien, insbesondere 1,3- Butadien oder Isopren und/oder ein Gemisch enthaltend mindestens eines der genannten Comonomere, mit Methylmethacrylat polymerisiert. Die vorgenannte Molmasse des Polymethylmethacrylat-Polymers entspricht dem Zahlenmittel der Molmasse Mₙ, diese kann nach der dem Fachmann geläufigen Methode der GPC-Analyse ermittelt werden.

Nachfolgend wird das pulverförmige Polymethylmethacrylat-Copolymer oder Gemische umfassend Polymethylmethacrylat-Copolymere zusammengefasst als pulverförmiges Polymethylmethacrylat-Copolymer bezeichnet, so dass unter dem Copolymer auch Gemische umfassend mindestens ein Polymethylmethacrylat-Copolymer gemeint sind.

Es ist besonders bevorzugt, wenn das Gewichtsverhältnis des Polyacrylats (ii), des Röntgenopakers und des Polymerisationsinitiators zum radikalisch polymerisierbaren Monomer (i) und optional zum Polymerisationsbeschleuniger von etwa 2,0 bis 3,4 zu 1,0 beträgt. Die genaue Einstellung dieses Gewichtsverhältnisses zur Herstellung des polymerisierbaren Knochenzementes erlaubt die spezifische Einstellung der initialen Viskosität des erhältlichen Knochenzementteiges.

Beschrieben wird somit ein polymerisierbarer Knochenzement, wobei der Knochenzement vorliegt als a) niedrigviskoser Knochenzement, insbesondere mit einer Klebfreiheit nach ISO5833 nach größer 3,0 Minuten nach dem Vermischen, oder b) mittelviskoser Knochenzement, insbesondere mit einer Klebfreiheit nach ISO5833 nach von größer gleich 1,5 bis 3,0 Minuten nach dem Vermischen, oder c) hochviskoser Knochenzement, insbesondere mit einer Klebfreiheit nach ISO5833 nach größer gleich 1,0 bis kleiner 1,5 Minuten nach dem Vermischen.

Die initiale Viskosität des Knochenzementes wird definiert über den Zeitraum ab der Vermischung der Komponenten bis zur Klebfreiheit des Knochenzementes. So wird die initiale Viskosität eines Zementes mit einer Klebfreiheit nach ISO5833 nach größer 3,0 Minuten als niedrigviskos eingestuft, tritt die Klebfreiheit nach ISO5833 nach größer gleich 1,5 bis 3,0 Minuten nach dem Vermischen ein, wird die initiale Viskosität als mittelviskos eingestuft und bei einer Klebfreiheit nach ISO5833 nach größer gleich 1,0 bis 1,5 Minuten nach dem Vermischen ist die initiale Viskosität hochviskos.

Beim Vermischen der Pulverkomponente mit der Monomerkomponente reagiert der Polymerisationsbeschleuniger, insbesondere N,N-Dimethyl-p-toluidin, mit dem Polymerisationsinitiator Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs bis dieser erstarrt.

Der Knochenzement erlaubt überraschenderweise eine Einstellung der Biegefestigkeit [MPa], des Biegemoduls [MPa] und/oder der Druckfestigkeit [MPa] der auspolymerisierten, gehärteten Knochenzemente, die mit einem Gewichtsverhältnis von 2,0 bis 3,4 zu 1,0 der Komponente A zur Komponente B gemischt wurden, gemessen nach ISO5833, der Biegefestigkeit von 78 bis 90 MPa, insbesondere von 79 bis 88 MPa, und/oder des Biegemoduls von 3100 bis 3700 MPa, insbesondere von 3150 bis 3600 MPa und/oder der Druckfestigkeit von 100 bis 113 MPa, insbesondere von 105 bis 114 MPa. Mit der Zusammensetzung können somit über einen weiten Bereich Knochenzemente hergestellt werden, die qualitativ deutlich über den Anforderungen der ISO5833 liegen.

Zur Herstellung der universal einsetzbaren Komponente A bzw. der Zusammensetzung umfassend die Komponenten (ii), (iii) und (iv) wird ein spezifischer Gehalt an Polymerisationsinitiator der Komponenten A oder in der vorgenannten Zusammensetzung eingestellt. Daher ist es besonders bevorzugt, wenn in der Zusammensetzung umfassend die Komponenten (ii), (iii) und (iv) oder der Komponente A der Gehalt an Polymerisationsinitiator von 1,0 bis 2,5 Gew.-% beträgt, insbesondere 1,0 bis 2,5 Gew.-% Dibenzoylperoxid, insbesondere in Bezug auf 100 Gew.-% bezogen auf die Komponenten A oder die Komponenten (ii), (iii) und (iv).

Nach weiteren Ausführungsvarianten kann das radikalisch polymerisierbare Monomer umfassend mindestens Methylmethacrylat sowie mindestens ein weiteres Monomer ausgewählt sein aus einem Alkyl-2-acrylsäurealkylester, Aryl-2-acrylsäurealkylester, Arylalkyl-2-acrylsäurealkylester, jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere,

Entsprechend einer besonders bevorzugten Ausführungsform umfasst eine Zusammensetzung zur Verwendung als polymerisierbarer Knochenzement oder zur Verwendung in dem Verfahren, insbesondere umfassend eine pharmazeutisch wirksame Substanz, zwei Komponenten A und B, wobei Komponente A als Pulver vorliegt und umfasst,
(a.1) 75 bis 85 Gew.-% mindestens eines pulverförmigen Polymethylmethacrylat-Copolymers oder eines Gemisches umfassend mindestens ein Polymethylmethacrylat-Copolymer ausgewählt aus einem partikulären Polymethylmethacrylat-Copolymer mit einer Molmasse Mn größer gleich 200.000 g/mol, insbesondere bis 1.000.000 g/mol, vorzugsweise bis 800.000 g/mol, besonders bevorzugt bis 300.000 g/mol, wobei das Polymethylmethacrylat-Copolymer erhältlich ist durch Polymerisation eines Gemisches von größer gleich 90,0 Gewichtsprozent Methylmethacrylat und kleiner gleich 10,0 Gewichtsprozent eines oder mehrerer Comonomere, die nicht Methylmethacrylat entsprechen, und die Gesamtzusammensetzung in Bezug auf dieses Gemisch 100 Gew.-% beträgt,
(a.2) 10 bis 20 Gew.-%, insbesondere 12 bis 16 Gew.-% mindestens eines pulverförmigen Röntgenopakers, insbesondere Zirkoniumdioxid und
(a.3) 1,0 bis 2,5 Gew.-% mindestens eines Polymerisationsinitiators, insbesondere Dibenzoylperoxid;
(a.4) 0,0 bis 10 Gew.-% mindestens einer pharmazeutisch wirksamen Substanz, insbesondere von 0,5 bis 5,0 Gew.-%; wobei insbesondere die pharmazeutisch wirksame Substanz mindestens ein Antibiotikum, wie ein Aminoglycosidantibiotikum, vorzugsweise ein pharmazeutisch wirksames Salz von Gentamicin, wie Gentamicinsulfat, Tobramycin, Vancomycin, Clindamycin, Erythromycin, Colistin und/oder deren pharmakologisch verträglichen Salze ist,
wobei die Gesamtzusammensetzung der Komponenten A 100 Gew.-% beträgt, und Komponente B als Flüssigkeit vorliegt und umfasst
(b.1) 95 bis 99,9 Gew.-%, insbesondere 97,5 bis 99 Gew.-% mindestens ein radikalisch polymerisierbares Monomer, insbesondere Methylmethacrylat,
(b.2) 0,1 bis 5 Gew.-%, insbesondere 1,0 bis 2,5 Gew.-%, mindestens einen Polymerisationsbeschleuniger, insbesondere ein aromatisches Amin, bevorzugt N,N-Dimethyl-p-toluidin, und
(b.3) 0 bis 2,0 Gew.-% mindestens einen Stabilisator, wie Hydrochinon, und (b.4) optional ein Gehalt an einem Farbstoff, wie Chlorophyllin E141, insbesondere 0 bis 100 ppm, bezogen auf das Gewicht.
wobei die Gesamtzusammensetzung der Komponenten B 100 Gew.-% beträgt, und wobei insbesondere das Gewichtsverhältnis der Komponente A umfassend das pulverförmige Polymethylmethacrylat-Copolymer oder ein Gemisch umfassend mindestens ein Polymethylmethacrylat-Copolymer, bevorzugt ein Gemisch von Polymethylmethacrylat-Copolymeren, zur Komponente B umfassend das radikalisch polymerisierbare Monomer von etwa 2,0 bis 3,4 zu 1,0 beträgt, besonders bevorzugt entspricht das Gewichtsverhältnis a), b) oder c).

Als pharmazeutisch wirksame Substanz kann die Komponente A mindestens ein Antibiotikum, Antimykotikum, Antiseptikum, Antiphlogistikum, mindestens einen Wachstumsfaktor und mindestens ein Bisphosphonat enthalten. Bevorzugte Antibiotika umfassen Gentamicin, Tobramycin, Amikacin, Vancomycin, Teicoplanin, Ramoplanin, Dalbavancin, Daptomycin, Fosfomycin, Clindamycin und/oder Lincomycin. Dabei sind Amphotericin B und Caspofungin als Antimykotika bevorzugt.

Entsprechend einer weiteren bevorzugten Ausführungsform kann die Partikelgröße der pulverförmigen Polymethylmethacrylat-Copolymer Partikel kleiner gleich 100 µm sein, insbesondere ist die Partikelgröße d₉₉ der Partikel kleiner gleich 100 µm, insbesondere von kleiner 100 µm bis 1 µm.

Gemäß einer weiteren bevorzugten Ausführungsform kann die Zusammensetzung mindestens ein Monomer mit Haftgruppen beinhalten. Bei der Haftgruppe kann es sich beispielsweise um eine Amid-Gruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Entsprechend einer besonders bevorzugten Alternative weist die Zusammensetzung, insbesondere vor der Polymerisation als pulverförmige Komponente A mindestens ein Polymethylmethacrylat-Polymer, einen Röntgenopaker und Dibenzoylperoxid auf und enthält separat als flüssige Komponente B polymerisierbares Monomer, Methylmethacrylat, einen Stabilisator und mindestens ein aromatisches Amin, wobei die Komponente A umfasst
a) mindestens ein partikuläres Polymethylmethacrylat-Copolymer mit einer Molmasse Mn größer 200.000 g/mol bis 1.000.000 g/mol, wobei das Polymethylmethacrylat-Copolymer durch Polymerisation eines Gemisches von größer gleich 90,0 Gewichtsprozent Methylmethacrylat und kleiner gleich 10,0 Gewichtsprozent eines oder mehrerer Comonomere hergestellt wurde, und
b) 1,0 bis 2,5 Gew.-% Dibenzoylperoxid,
wobei das Gewichtsverhältnis von Komponente A zu Komponente B von 2,0 bis 3,4 zu 1,0 variiert wird, um die initiale Viskosität des Zementteigs zu steuern, der durch Vermischung des Zementpulvers mit der Monomerflüssigkeit gebildet wird.

Ebenso beschrieben wird ein Verfahren zur Herstellung eines Knochenzementteiges mit variabler initialer Viskosität. Nach dem Verfahren werden die Komponente A bzw. die Komponenten (ii), (iii) und (iv) mit der Komponente B bzw. der Komponente (i) und mindestens ein Polymerisationsinitiator im Gewichtsverhältnis von 2,0 bis 3,4 zu 1,0 miteinander vermischt, um die initiale Viskosität des erhältlichen Polymethylmethacrylat-Knochenzements einzustellen. In dem Verfahren beträgt das Gewichtsverhältnis beim Mischen alternativ a) kleiner gleich 2,2 zu 1,0, oder b) von 2,2 bis kleiner 3,3 zu 1,0, oder c) von größer gleich 3,3 zu 1,0, um polymerisierbare Knochenzemente herzustellen.

Die Variation der Menge an Monomerflüssigkeit kann über eine geeignete Dosiereinrichtung, wie zum Beispiel eine automatische Pipette, oder auch durch geeignete Full-Prepacked-Mischsysteme, wie in der Anmeldung DE102015106899.0 (04.05.2015 Priorität beim DPMA) beschrieben, erfolgen (nicht erfindungsgemäß). Dieses Full-Prepacked-Mischsystem verfügt über eine Vorrichtung zur einstellbaren Dosierung der Menge an Monomerflüssigkeit mit der das Gewichtsverhältnis von Zementpulver zu Monomerflüssigkeit variiert werden kann.

Ferner beschrieben wird ein Verfahren zur Herstellung eines polymerisierbaren Knochenzementes, indem zwei Komponenten A und B miteinander gemischt werden, wobei Komponente A als Pulver vorliegt und umfasst (a.1) mindestens ein pulverförmiges Polymethylmethacrylat-Copolymer, wobei das Polymethylmethacrylat-Copolymer umfasst mindestens ein partikuläres Polymethylmethacrylat-Copolymer mit einer Molmasse Mn größer gleich 200.000 g/mol bis 1.000.000 g/mol, wobei das Polymethylmethacrylat-Copolymer erhältlich ist durch Polymerisation eines Gemisches von größer gleich 90,0 Gewichtsprozent Methylmethacrylat und kleiner gleich 10,0 Gewichtsprozent eines oder mehrerer Comonomere, und die Gesamtzusammensetzung in Bezug auf dieses Gemisch 100 Gew.-% beträgt, (a.2) mindestens einen pulverförmigen Röntgenopaker, und (a.3) mindestens einen Polymerisationsinitiator; und
Komponente B als Flüssigkeit vorliegt und umfasst (b.1) mindestens ein radikalisch polymerisierbares Monomer, insbesondere mindestens Methylmethacrylat, (b.2) mindestens einen Polymerisationsbeschleuniger und (b.3) mindestens einen Stabilisator, wobei das Gewichtsverhältnis der Komponente A umfassend das Polymethylmethacrylat-Copolymer zur Komponente B umfassend das radikalisch polymerisierbare Monomer von etwa 2,0 bis 3,4 zu 1,0 beträgt, wobei insbesondere Komponente A von 1,0 bis 2,5 Gew.-% mindestens einen Polymerisationsinitiator insbesondere Dibenzoylperoxid in Bezug auf die Gesamtzusammensetzung der Komponente A von 100 Gew.-% aufweist. Die Komponenten A und B liegen immer als jeweilige Gesamtzusammensetzung von 100 Gew.-% vor.

Nach besonders bevorzugten Verfahrensvarianten werden die Komponente A und die Komponente B im Gewichtsverhältnis von a) kleiner 2,2 zu 1,0 gemischt, oder b) von 2,2 bis kleiner 3,3 zu 1,0, insbesondere von größer 2,2 bis 3,2 zu 1,0, oder c) von größer gleich 3,3 zu 1,0 gemischt, insbesondere von 3,3 bis 3,4 zu 1,0, um polymerisierbare Knochenzemente herzustellen und, insbesondere über das Mischungsverhältnis die initiale Viskosität der polymerisierbaren Knochenzemente zu steuern.

Entsprechend einer weiteren besonders bevorzugten Variante können die Komponente A und die Komponente B im Gewichtsverhältnis von a) kleiner 2,2 zu 1,0 gemischt werden, insbesondere von 2,0 bis kleiner 2,2 zu 1,0, um niedrigviskose Knochenzemente mit einer Klebfreiheit nach ISO5833 nach größer 3,0 Minuten nach dem Vermischen herzustellen, oder b) von 2,2 bis kleiner 3,3 zu 1,0 gemischt werden, insbesondere von größer 2,2 bis 3,2 zu 1,0 gemischt werden, um mittelviskose Knochenzemente mit einer Klebfreiheit nach ISO5833 nach von größer gleich 1,5 bis 3,0 Minuten nach dem Vermischen herzustellen, oder c) von größer gleich 3,3 zu 1,0 gemischt werden, insbesondere von 3,3 bis 3,4 zu 1,0, um hochviskose Knochenzemente mit einer Klebfreiheit nach ISO5833 nach größer gleich 1,0 bis kleiner 1,5 Minuten nach dem Vermischen herzustellen.

Es ist bevorzugt, wenn in dem Verfahren die Dosierung der Komponente A und der Komponente B im Gewichtsverhältnis entsprechend a), b) oder c) über die Dosierung des jeweiligen auf das Gewichtsverhältnis umgerechneten Volumens, bspw. in Millilitern oder Litern, der Komponenten A und B erfolgt.

Nach einer weiteren bevorzugten Alternative wird ein Verfahren beschrieben, in dem eine definierten Menge der Komponente A in Gramm zur Einstellung des Gewichtsverhältnisses a), b) oder c) oder eines dieser Menge entsprechenden Volumens der Komponente A im Innenraum einer Kartusche vorgelegt wird, wobei die Kartusche an ihrem einen Ende einen Kartuschenanschluss aufweist und an ihrem anderen, gegenüberliegenden Ende einen Austragskolben aufweist, wobei durch eine Durchführung in dem Austragskolben eine Mischstange an eine Mischeinrichtung im Inneren der Kartusche anbringbar ist oder angeordnet ist, wobei die Mischeinrichtung von außen durch Bewegen der Mischstange entlang einer Achse des Innenraumes bedienbar ist, wobei optional der Kartusche eine Verbindungsleitung zugeordnet ist, und, die Komponente B in einem Einweg Monomer-Behälter vorgelegt wird. Die Verbindungsleitung kann mittelbar oder unmittelbar dem Kartuschenanschluss zugeordnet sein.

Des Weiteren umfasst das Verfahren bevorzugt, die Schritte: Überführen einer definierten Menge der Komponente B in Gramm zur Einstellung des Gewichtsverhältnisses a), b) oder c) oder eines diesem entsprechenden Volumens der Komponente B aus dem Einweg Monomer-Behälter in die Kartusche, insbesondere über eine Dosiereinrichtung und optional eine Verbindungsleitung, und Mischen der Komponente A und B.

Ferner werden in dem Verfahren durch das Mischen der Komponente A und der Komponente B im Gewichtsverhältnis von a) kleiner 2,2 zu 1,0 niedrigviskose Knochenzemente mit einer Klebfreiheit nach ISO5833 nach größer 3,0 Minuten nach dem Vermischen erhalten, oder b) von 2,2 bis kleiner 3,3 zu 1,0 mittelviskose Knochenzemente mit einer Klebfreiheit nach ISO5833 nach von größer gleich 1,5 bis 3,0 Minuten nach dem Vermischen erhalten, oder c) von größer gleich 3,3 zu 1,0 hochviskose Knochenzemente mit einer Klebfreiheit nach ISO5833 nach größer gleich 1,0 bis kleiner 1,5 Minuten nach dem Vermischen erhalten.

Nach einer weiteren, besonders bevorzugten Verfahrensvariante werden die Komponente A und die Komponente B im Innenraum mit einer Mischeinrichtung gemischt, indem die Mischeinrichtung durch Bewegen einer drehbar und in Längsrichtung verschiebbar in den Innenraum der Kartusche geführten Mischstange bedient wird, wobei bevorzugt nach dem Mischen die Mischstange bis zum Anschlag aus dem Innenraum der Kartusche herausgezogen wird und besonders bevorzugt die Mischstange nach dem Herausziehen bis zum Anschlag an einer Sollbruchstelle abgebrochen wird.

Entsprechend einer weiteren, besonders bevorzugten Verfahrensvariante des Verfahrens wird die Kartusche mit dem niedrigviskosen, mittelviskosen oder hochviskosen polymerisierbaren Knochenzement von der Verbindungsleitung und optional einer Kartuschenhalterung gelöst und der Knochenzement wird durch Vortreiben eines Austragskolbens, der axial beweglich in der Kartusche gelagert ist und der den Innenraum der Kartusche auf einer Seite begrenzt, aus dem Innenraum der Kartusche ausgetragen.

Gegenstand der Erfindung ist ein Kit zur Herstellung von polymerisierbaren Knochenzementen, umfassend die Komponenten A und die Komponente B wobei die Komponente A als Pulver vorliegt und umfasst
(a.1) mindestens ein pulverförmiges Polymethylmethacrylat-Copolymer, wobei das pulverförmige Polymethylmethacrylat-Copolymer mindestens ein partikuläres Polymethylmethacrylat-Copolymer mit einer Molmasse Mn größer gleich 200.000 g/mol bis 1.000.000 g/mol umfasst, und das Polymethylmethacrylat-Copolymer erhältlich ist durch Polymerisation eines Gemisches von größer gleich 90,0 Gew.-% Methylmethacrylat und kleiner gleich 10,0 Gew.-% bis größer gleich 1 Gew.-% eines oder mehrerer Comonomere, und die Gesamtzusammensetzung in Bezug auf dieses Gemisch 100 Gew.-% beträgt,
(a.2) mindestens einen pulverförmigen Röntgenopaker, und
(a.3) mindestens einen Polymerisationsinitiator; und
Komponente B als Flüssigkeit vorliegt und umfasst
(b.1) mindestens ein radikalisch polymerisierbares Monomer,
(b.2) optional mindestens einen Polymerisationsbeschleuniger und
(b.3) optional mindestens einen Stabilisator, und
wobei die Komponente A im Innenraum (13) einer Kartusche (1) vorliegt, wobei die Kartusche (1) an ihrem einen Ende einen Kartuschenanschluss mit Innengewinde (8) und an ihrem anderen, gegenüberliegenden Ende einen Austragskolben (2) aufweist, wobei durch eine Durchführung in dem Austragskolben (2) eine Mischstange (4) an eine Mischeinrichtung im Inneren (13) der Kartusche anbringbar ist oder angeordnet ist, wobei die Mischeinrichtung von außen durch Bewegen der Mischstange (4) entlang einer Achse des Innenraumes (13) bedienbar ist, und wobei die Komponente B in einem Einweg oder mehrfach verwendbaren Monomer-Behälter enthalten ist, wobei die Komponente B in einer Glasampulle mit abbrechbarem Ampullenkopf vorliegt, wobei die Glasampulle, die Kartusche (1) und/oder eine Dosiereinrichtung, die zwischen dem Monomer-Behälter und der Kartusche angeordnet ist, Markierungen aufweisen, wobei den Markierungen ist die Zugabemenge der Komponente B zur Einstellung des Gewichtsverhältnisses oder das dem Gewichtsverhältnis entsprechenden Volumenverhältnis der Komponente A umfassend das Polymethylmethacrylat-Copolymer zur Komponente B umfassend das radikalisch polymerisierbare Monomer von etwa 2,0 bis 3,4 zu 1,0 in Skalenteilen der Markierung ablesbar ist. Alternativ oder zusätzlich können die Markierungen vorzugsweise als Rasterungen bspw. innen in der Kartusche und/oder an der Dosiereinrichtung vorliegen, denen bspw. außen an der Kartusche entsprechende Markierungen, bspw. als Beschriftung mit den Angaben auf das Gewichtsverhältnis, Volumenverhältnis oder die eingestellte Viskosität zugeordnet sind.

Besonders bevorzugt ist die Dosiereinrichtung dem Monomer-Behälter zugeordnet sowie der Verbindungsleitung der Kartusche, so dass das aus dem Monomer-Behälter austretendes Monomer in die Dosiereinrichtung überführt werden kann und von dort entsprechend dem gewünschten Mischungsverhältnis in die Kartusche überführt werden kann. Die Dosiereinrichtung umfasst vorzugsweise einen hohlzylindrischen Körper mit einem axial verschiebbaren Kolben. In den Hohlzylinder kann das Monomer einlaufen oder aufgenommen werden. Die Überführung des Monomers und dessen Dosierung in die Kartusche kann über Rasterungen im Hohlzylinder und/oder dem axial verschiebbaren Kolben erfolgen.

Besonders bevorzugt sind an der Dosiereinrichtung, Rasterungen, und/oder in einem Kartuschenfenster Markierungen vorgesehen, die eine Zugabe als Gewichtsverhältnis der Komponente B zur Einstellung des Gewichtsverhältnisses der Komponente A umfassend das Polymethylmethacrylat-Copolymer zur Komponente B anzeigen. Die Kartusche weist erfindungsgemäß eine Mischkammer auf.

Nach einer weiteren besonders bevorzugten Alternative ist der Austragskolben 2 für die Komponente A undurchlässig, vorzugsweise ist in dem Austragskolben 2 ein Porenfilter angeordnet, der für Gas durchlässig und für die Komponente B undurchlässig ist.

Beschrieben wird auch ein polymerisierbarer Knochenzement erhältlich durch Vermischen und Polymerisieren der Komponenten A und B, nach dem Verfahren oder Mischen der Komponenten A und B des Kits oder durch Mischen der Komponenten (i), (ii), (iii), (iv) sowie optional eines Polymerisationsbeschleunigers.

Nach einer besonders bevorzugten Ausführungsform wird die Verwendung einer Zusammensetzung umfassend die Komponente A und B beschrieben, oder die Verwendung der Zusammensetzung erhältlich nach dem Verfahren oder des Kits zur Herstellung, insbesondere zur variablen Einstellung der initialen Viskosität von Knochenzementen von niedrig über mittel bis hochviskos, insbesondere von
a) niedrigviskosen Knochenzementen, indem die Komponente A und die Komponente B im Gewichtsverhältnis von kleiner 2,2 zu 1,0 gemischt werden, wobei die Knochenzemente eine Klebfreiheit nach ISO5833 nach größer 3,0 Minuten nach dem Vermischen aufweisen, oder
b) mittelviskosen Knochenzementen, indem die Komponente A und die Komponente B im Gewichtsverhältnis von 2,2 bis kleiner 3,3 zu 1,0 gemischt werden, wobei die Knochenzemente eine Klebfreiheit nach ISO5833 nach von größer gleich 1,5 bis 3,0 Minuten nach dem Vermischen aufweisen, oder
c) hochviskosen Knochenzementen, indem die Komponente A und die Komponente B im Gewichtsverhältnis von größer gleich 3,3 gemischt werden, wobei die Knochenzemente mit einer Klebfreiheit nach ISO5833 nach größer gleich 1,0 bis kleiner 1,5 Minuten nach dem Vermischen aufweisen.

Nach einer weiteren Ausführungsvariante wird ein polymerisierter, gehärteter Knochenzement beschrieben, insbesondere in Form eines dreidimensionalen Formkörpers, vorzugsweise eines chirurgischen Implantats oder Teils davon. Ferner beschrieben wird ein Implantat zur Verwendung als chirurgisches Implantat oder Teil eines Implantates, Revisionsimplantat, Schraube, Nagel, chirurgische Platte, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie, als Femur- und Tibia-Komponenten von Kniegelenkendoprothesen, als Schultergelenkendoprothese oder als Trägermaterial für eine lokale Freisetzung von pharmazeutisch wirksamen Substanzen. Ebenso beschrieben werden die vorgenannten Implantate oder Erzeugnisse. Beschrieben wird nach einer Alternative eine Zusammensetzung, ein Kit oder ein chirurgisches Implantat bestehend aus dem gehärteten Knochenzement zur Verwendung bei der Augmentation von osteoporotischen Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie, als Trägermaterial für eine lokale Freisetzung von pharmazeutisch wirksamen Substanzen.

Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen auch Farblacke, wie zum Beispiel Farblack Grün, das Aluminiumsalz einer Mischung aus E104 und E132.

Röntgenopaker können vorzugsweise aus den Gruppen ausgewählt sein die umfassen Metalloxide, wie insbesondere Zirkoniumdioxid, Bariumsulfat, toxikologisch unbedenkliche Schwermetallpartikel, wie zum Beispiel Tantal, Ferrit, Magnetit (ggf. auch supramagnetisches Magnetit) und aus biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Der Gehalt an Röntgenopaker, insbesondere die Konzentration an Zirkoniumdioxid, in der Komponente A kann jeweils voneinander unabhängig beispielsweise in einem Bereich von 3 bis 30 Gew.-% in Bezug auf die jeweilige Gesamtzusammensetzung liegen.

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden Verbindungen verstanden, die wenigstens eine Peroxogruppe (-O-O-) enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten und 1,3,5-trisubstituierten Barbituraten. Gemäß einer besonderen Ausgestaltung ist das Barbitursäurederivat ausgewählt aus der Gruppe bestehend aus 1,5-disubstituierten Barbituraten und 1,3,5-trisubstituierten Barbituraten.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf. Erfindungsgemäß bevorzugt sind Barbiturate, die an Position 1 und an Position 5 jeweils einen Substituenten oder an den Positionen 1, 3 und 5 jeweils einen Substituenten tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethylbarbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt. Bevorzugte Schwermetallverbindungen sind ausgewählt aus der Gruppe bestehend Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform der Zusammensetzung ist der Polymerisationsbeschleuniger ausgewählt aus der Gruppe bestehend aus aromatischen Aminen, wie insbesondere N,N-Dimethyl-p-toluidin, N,N-Bis-hydroxyethyl-p-toluidin, N,N-Dimethyl-anilin, Phthalimid, Succinimid, Pyromelithsäurediimid und einer Mischung aus mindestens zwei davon.

Eine weitere vorteilhafte Ausgestaltung umfasst eine Verwendung von Kombinationen aus Schwermetallsalzen und mindestens einem Vertreter aus der Gruppe umfassend aus N,N-Dimethyl-p-toluidin, N,N-Bis-hydroxyethyl-p-toluidin, N,N-Dimethyl-anilin, als Polymerisationsbeschleuniger. Dabei werden Zweifachkombinationen und auch Dreifachkombinationen von unterschiedlichen Polymerisationsbeschleunigern im Rahmen der Erfindung offenbart.

Eine vorteilhafte Ausgestaltung besteht darin, dass gegebenenfalls wenigstens ein Copolymerisationsbeschleuniger in der erfindungsgemäßen Zusammensetzung oder in einer der Komponenten A oder B enthalten ist, wobei als Copolymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethyl-anilin als Co-Akzeleratoren bevorzugt sind.

Der Polymethylmethacrylat-Knochenzement kann für die mechanische Fixierung von primären Gelenkendoprothesen und auch zur Verankerung von Revisions-Gelenkendoprothesen verwendet werden. Ebenfalls kann der Polymethylmethacrylat-Knochenzement zur Herstellung von Spacern und von lokalen Wirkstoffträgern eingesetzt werden. Es ist auch möglich, mit dem Polymethylmethacrylat-Knochenzement Knochendefekte am Schädelknochen abzudecken.

Für die Zemente der Beispiele (a-j) wurde ein durch Suspensionspolymerisation hergestelltes Polymethylmethacrylat-co-methylmethacrylat mit einer zahlenmittleren Molmasse größer 200.000 g/ml verwendet. Diese Copolymer wurde aus einem Gemisch von Methylmethacrylat und Methylacrylat hergestellt, wobei der Methylmethacrylat-Gehalt größer 90 Gewichtsprozent und der Methylacrylat-Gehalt kleiner 10 Gewichtsprozent war. Es wurde die Siebfraktion kleiner 100 µm der Polymer-Beads des Polymethylmethacrylat-co-methylmethacrylats verwendet. Als Initiator wurde handelsübliches mit Wasser phlegmatisiertes Dibenzoylperoxid verwendet. Handelsübliches Zirkoniumdioxid wurde als Röntgenopaker eingesetzt.

Zusammensetzung der Komponente A als Zementpulver 1:
15,0 Gew.-% Zirkoniumdioxid
2,0 Gew.-% Dibenzoylperoxid
83,0 Gew.-% Polymethylmethacrylat-co-methylmethacrylat

Die Komponente B synonym Monomerflüssigkeit hatte folgende Zusammensetzung:
98 Gew.-% Methylmethacrylat, 2,0 Gew.-% N,N-Dimethyl-p-toluidin, Spuren von Chlorophyllin E141, stabilisiert mit ∼ 40 ppm Hydrochinon

Die Prüfung der Verarbeitungseigenschaften der Zemente (a-e) wurde gemäss ISO5833 voraenommen.

| Zement | Gewichtsverhältnis Zementpulver zu Monomer-flüssigkeit | Mischzeit | Klebfreiheit [min] | Ende der Verarbeitung |
|---|---|---|---|---|
| a | 2,1 : 1,0 | 30 s | 3 min 0 s | 5 min 10 s |
| b | 2,4 : 1,0 | 30 s | 2 min 29 s | 4 min 40 s |
| c | 2,6 : 1,0 | 30 s | 2 min 10 s | 4 min 5 s |
| d | 2,9 : 1,0 | 30 s | 1 min 45 s | 3 min 58 s |
| e | 3,2 : 1,0 | 30 s | 1 min 35 s | 3 min 45 s |

Bei dem Zement (a) handelt es sich um einen niedrigviskosen Zement. Die Zemente (b), (c) und (d) sind als mittelviskos zu bewerten. Der Zement (e) ist als hochviskos einzustufen.

Es wurden für die Bestimmung der Biegefestigkeit und des Biegemoduls der Zemente (a-e) gemäß ISO 5833 streifenförmige Prüfkörper mit den Abmessungen 3,3 mm x 10,0 mm x 75 mm hergestellt. Für die Bestimmung der Druckfestigkeit wurden zylinderförmige Probekörper mit einem Durchmesser von 6 mm und mit einer Höhe von 10 mm gefertigt. Die Prüfung der Biegefestigkeit, des Biegemoduls und der Druckfestigkeit gemäß der ISO 5833 erfolgte mit einer Zwick-Universalprüfmaschine Z010.

| Zement | Gewichtsverhältnis Zementpulver zu Monomerflüssigkeit | Biegefestigkeit [MPa] | Biegemodul [MPa] | Druckfestigkeit [MPa] |
|---|---|---|---|---|
| a | 2,1 : 1,0 | 80,3 ± 1,2 | 3246 ± 94 | 107,1 ± 2,2 |
| b | 2,4 : 1,0 | 81,8 ± 1,3 | 3277 ± 107 | 109,6 ± 1,6 |
| c | 2,6 : 1,0 | 79,7 ± 0,8 | 3177 ± 55 | 113,3 ± 0,6 |
| d | 2,9 : 1,0 | 81,6 ± 1,6 | 3267 ± 68 | 112,1 ± 1,4 |
| e | 3,2 : 1,0 | 85,8 ± 2,2 | 3510 ± 162 | 112,6 ± 1,7 |

Die Norm ISO5833 fordert für eine Biegefestigkeit größer 50 MPa, ein Biegemodul größer 1800 MPa und eine Druckfestigkeit von größer 70 MPa. Die Zemente der Beispiele (a-e) erfüllen die Anforderungen der ISO5833 hinsichtlich der Biegefestigkeit, des Biegemoduls und der Druckfestigkeit.

Es wurde auch eine Gentamicin enthaltende Komponente A2 als Zementpulver 2 hergestellt.

Zusammensetzung des Zementpulvers 2 mit Zusatz von Gentamicinsulfat:
14,4 Gew.-% Zirkoniumdioxid
1,8 Gew.-% Dibenzoylperoxid
79,7 Gew.-% Polymethylmethacrylat-co-methylmethacrylat
4,1 Gew.-% Gentamicinsulfat (entspricht 2,5 Gew.-% Gentamicinbase)

Die Monomerflüssigkeit hatte folgende Zusammensetzung: 98 Gew.-% Methylmethacrylat, 2,0 Gew.-% N,N-Dimethyl-p-toluidin, Spuren von Chlorophyllin E141, stabilisiert mit ∼ 100 ppm Hydrochinon

Die Prüfung der Verarbeitungseigenschaften der Zemente a-e wurde gemäss ISO5833 vorgenommen.

| Zement | Gewichtsverhältnis von Zementpulver 2 zu Monomerflüssigkeit | Mischzeit | Klebfreiheit [min] | Ende der Verarbeitung |
|---|---|---|---|---|
| f | 2,2 : 1,0 | 30 s | 3 min 20 s | 5 min 32 s |
| g | 2,5 : 1,0 | 30 s | 2 min 40 s | 4 min 55 s |
| h | 2,7 : 1,0 | 30 s | 2 min 20 s | 4 min 30 s |
| i | 3,0 : 1,0 | 30 s | 1 min 58 s | 4 min 10 s |
| j | 3,3 : 1,0 | 30 s | 1 min 15 s | 3 min 50 s |

Bei dem Zement (f) handelt es sich um einen niedrigviskosen Zement. Die Zemente (g), (h) und (i) sind als mittelviskos zu bewerten. Der Zement (j) ist als hochviskos einzustufen.

Es wurden für die Bestimmung der Biegefestigkeit und des Biegemoduls der Zemente (f-j) gemäß ISO5833 streifenförmige Prüfkörper mit den Abmessungen 3,3 mm x 10,0 mm x 75 mm hergestellt. Für die Bestimmung der Druckfestigkeit wurden zylinderförmige Probekörper mit einem Durchmesser von 6 mm und mit einer Höhe von 10 mm gefertigt. Die Prüfung der Biegefestigkeit, des Biegemoduls und der Druckfestigkeit gemäß der ISO5833 erfolgte mit einer Zwick-Universalprüfmaschine Z010.

| Zement | Gewichtsverhältnis von Zementpulver 2 zu Monomerflüssigkeit | Biegefestigkeit [MPa] | Biegemodul [MPa] | Druckfestigkeit [MPa] |
|---|---|---|---|---|
| f | 2,2 : 1,0 | 66,6 ± 2,2 | 3092 ± 73 | 104,3 ± 1,1 |
| g | 2,5 : 1,0 | 68,8 ± 3,1 | 3204 ± 77 | 107,8 ± 1,4 |
| h | 2,7 : 1,0 | 74,3 ± 2,5 | 3740 ± 118 | 111,9 ± 1,4 |
| i | 3,0 : 1,0 | 74,2 ± 1,6 | 3627 ± 53 | 108,8 ± 3,7 |
| j | 3,3 : 1,0 | 72,9 ± 2,1 | 3585 ± 31 | 96,2 ± 3,2 |

Die Norm ISO5833 fordert für eine Biegefestigkeit größer 50 MPa, ein Biegemodul größer 1800 MPa und eine Druckfestigkeit von größer 70 MPa. Die Zemente der Beispiele (f-j) erfüllen die Anforderungen der ISO5833 hinsichtlich der Biegefestigkeit, des Biegemoduls und der Druckfestigkeit.

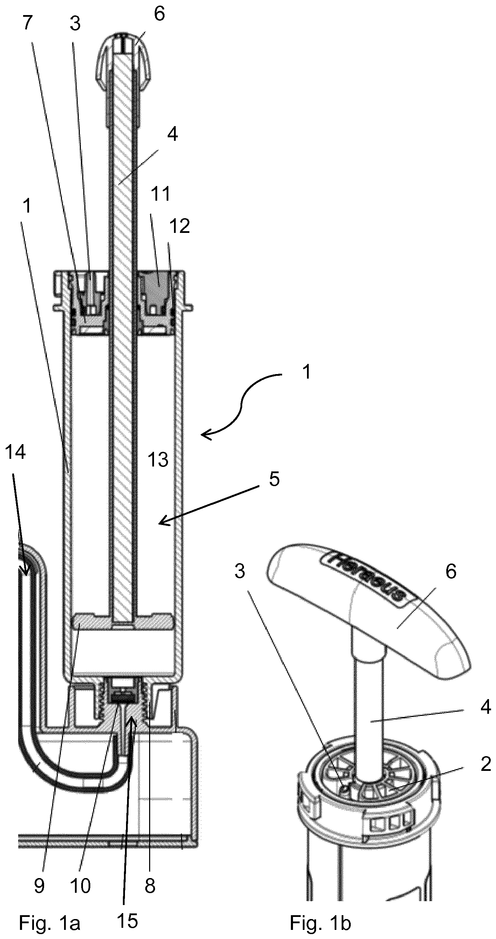

Die Figuren 1a und 1b stellen ein Kit umfassend eine Kartusche **1** und insbesondere einen Einweg Monomer-Behälter (Vial, nicht dargestellt) dar. Weitere Details zum Aufbau der Kartusche sind in Figur 1a und 1b zu erkennen. Das Innere der Kartusche **1** wird durch einen zylindrischen Innenraum **13** gebildet, in dem das Zementpulver enthalten ist. Zudem ist im Innenraum **13** der Kartusche **1** eine Mischeinrichtung **9** bestehend aus mehreren Mischflügeln **9** angeordnet, die an der Mischstange **4** befestigt ist und die über die Mischstange **4** im Innenraum **13** bewegt werden kann. In der Mischkammer **5** können durch Bewegung der Mischstange **4** die Komponenten A und B miteinander gemischt werden. Der Austragskolben **2** ist zweiteilig aufgebaut und besteht aus einem Sterilisationskolben **7** (in Figur 1a oberer Teil des Austragskolbens) und einem mit einer Dichtung **12** gegen die Innenwand der Innenraums **13** abgedichteten Dichtungskolben **11** (in Figur 1a unterer Teil des Austragskolbens). Der Dichtungskolben **11** weist eine gasdurchlässige aber für Pulver undurchlässige Porenscheibe auf, durch die der Innenraum **13** evakuierbar ist. Der Austragskolben **2** hat einen zylindrischen Außenumfang und schließt dicht mit den Wänden des Innenraums **13** ab. Der Kartusche **1**, insbesondere dem Kartuschenanschluss **8**, ist eine Verbindungsleitung **14** zugeordnet, durch die das Monomer vor dem Vermischen in die Kartusche **1** eingebracht werden kann.

Zwischen dem Monomer-Behälter (Vial) und der Verbindungsleitung **14** kann eine Dosiereinrichtung angeordnet sein, in die das aus dem Monomer-Behälter austretende Monomer überführt wird. Die Dosiereinrichtung umfasst vorzugsweise einen hohlzylindrischen Körper in dem ein axial verschiebbarer Kolben angeordnet ist. In den Hohlzylinder der Dosiereinrichtung mit kann das Monomer einlaufen oder aufgenommen werden. Durch definiertes Einschieben des axial verschiebbaren Kolbens in den Hohlzylinder umfassend das Monomer, wird die gewünschte Menge an Monomer für das erfindungsgemäße Mischungsverhältnis in der Kartusche eingestellt, indem diese Menge in die Kartusche überführt wird. Die Einstellung des Mischungsverhältnisses kann über Rasterungen im Hohlzylinder und/oder an dem axial verschiebbaren Kolben erfolgen.

Der Austragskolben **2** kann im Innenraum **13** in Richtung einer Austragsöffnung vorgetrieben werden, die auf der dem Austragskolben **2** gegenüberliegenden Seite des Innenraums **13** der Kartusche **1** angeordnet ist. Der gemischte Knochenzement kann aus dem Innenraum der Kartusche durch die Austragsöffnung und das Austragsrohr durch Einpressen des Austragskolbens **2** ausgetrieben und appliziert werden. Vor dem Austreiben und Applizieren wird die Kartusche **1** von der Verbindungsleitung **14** und optional einer Kartuschenhalterung **15** abgetrennt.

**Bezugszeichenliste: 1** Kartusche; **2** Austragskolben; **3** Vakuumanschluss; **4** Mischstange; **5** Mischkammer; **6** Griffstück; **7** Sterilisationskolben; **8** Kartuschenanschluss mit Innengewinde; **9** Mischflügel / Mischeinrichtung; **10** Pulverundurchlässiger und Flüssigkeitsdurchlässiger Filter; **11** Dichtungskolben; **12** Dichtung; **13** Innenraum; **14** Verbindungsleitung, **15** Kartuschenhalterung.

## Patentansprüche

1. Kit zur Herstellung von polymerisierbaren Knochenzementen, **dadurch gekennzeichnet, dass** es die Komponente A und die Komponente B umfasst, wobei Komponente A als Pulver vorliegt und umfasst
(a.1) mindestens ein pulverförmiges Polymethylmethacrylat-Copolymer, wobei das pulverförmige Polymethylmethacrylat-Copolymer mindestens ein partikuläres Polymethylmethacrylat-Copolymer mit einer Molmasse Mn größer gleich 200.000 g/mol bis 1.000.000 g/mol umfasst, und das Polymethylmethacrylat- Copolymer erhältlich ist durch Polymerisation eines Gemisches von größer gleich 90,0 Gew.-% Methylmethacrylat und kleiner gleich 10,0 Gew.-% bis größer gleich 1 Gew.-% eines oder mehrerer Comonomere, und die Gesamtzusammensetzung in Bezug auf dieses Gemisch 100 Gew.-% beträgt,
(a.2) mindestens einen pulverförmigen Röntgenopaker, und
(a.3) mindestens einen Polymerisationsinitiator; und
Komponente B als Flüssigkeit vorliegt und umfasst
(b.1) mindestens ein radikalisch polymerisierbares Monomer,
(b.2) optional mindestens einen Polymerisationsbeschleuniger und
(b.3) optional mindestens einen Stabilisator, und
wobei die Komponente A im Innenraum (13) einer Kartusche (1) vorliegt, wobei die Kartusche (1) an ihrem einen Ende einen Kartuschenanschluss mit Innengewinde (8) und an ihrem anderen, gegenüberliegenden Ende einen Austragskolben (2) aufweist, wobei durch eine Durchführung in dem Austragskolben (2) eine Mischstange (4) an eine Mischeinrichtung im Inneren (13) der Kartusche anbringbar ist oder angeordnet ist, wobei die Mischeinrichtung von außen durch Bewegen der Mischstange (4) entlang einer Achse des Innenraumes (13) bedienbar ist, und,
wobei die Komponente B in einem Einweg oder mehrfach verwendbaren Monomer-Behälter enthalten ist, wobei die Komponente B in einer Glasampulle mit abbrechbarem Ampullenkopf vorliegt,
wobei die Glasampulle, die Kartusche (1) und/oder eine Dosiereinrichtung, die zwischen dem Monomer-Behälter und der Kartusche angeordnet ist, Markierungen aufweisen, wobei an den Markierungen die Zugabemenge der Komponente B zur Einstellung des Gewichtsverhältnisses oder das dem Gewichtsverhältnis entsprechenden Volumenverhältnis der Komponente A umfassend das Polymethylmethacrylat-Copolymer zur Komponente B umfassend das radikalisch polymerisierbare Monomer von etwa 2,0 bis 3,4 zu 1,0 in Skalenteilen der Markierung ablesbar ist.

2. Verfahren zur Herstellung eines polymerisierbaren Knochenzementes unter Verwendung eines Kits nach Anspruch 1, indem zwei Komponenten A und B miteinander gemischt werden, wobei
Komponente B als Flüssigkeit vorliegt und umfasst
(b.1) mindestens ein radikalisch polymerisierbares Monomer,
(b.2) mindestens einen Polymerisationsbeschleuniger und
(b.3) mindestens einen Stabilisator,
Komponente A als Pulver vorliegt und umfasst
(a.1) mindestens ein in (b.1) lösliches pulverförmiges Polymethylmethacrylat-Copolymer oder ein pulverförmiges Gemisch umfassend in (b.1) lösliche Polymethylmethacrylat-Copolymere, wobei das Polymethylmethacrylat-Copolymer umfasst mindestens ein partikuläres Polymethylmethacrylat-Copolymer mit einer Molmasse größer gleich 200.000 g/mol und das Polymethylmethacrylat-Copolymer erhältlich ist durch Polymerisation eines Gemisches von größer gleich 90,0 Gew.-% Methylmethacrylat und kleiner gleich 10,0 Gew.-% bis größer gleich 1 Gew.-% eines oder mehrerer Comonomere, und die Gesamtzusammensetzung des Polymethylmethacrylat-Copolymers in Bezug auf dieses Gemisch 100 Gew.-% beträgt,
(a.2) mindestens einen pulverförmigen Röntgenopaker, und
(a.3) mindestens einen Polymerisationsinitiator; und
wobei das Gewichtsverhältnis der Komponente A umfassend das Polymethylmethacrylat-Copolymer zur Komponente B umfassend das radikalisch polymerisierbare Monomer von etwa 2,0 bis 3,4 zu 1,0 beträgt und
die Komponenten A und B im Gewichtsverhältnis ausgewählt aus
a) kleiner 2,2 zu 1,0 oder
b) von 2,2 bis kleiner 3,3 zu 1,0, oder
c) von größer gleich 3,3 zu 1,0 gemischt werden.

3. Verfahren nach Anspruch 2, **dadurch gekennzeichnet, dass** Komponente A von 1,0 bis 2,5 Gew.-% mindestens einen Polymerisationsinitiator, vorzugsweise Dibenzoylperoxid in Bezug auf die Gesamtzusammensetzung der Komponente A von 100 Gew.-% aufweist.

4. Verfahren nach Anspruch 3, **dadurch gekennzeichnet, dass** die Komponente A und die Komponente B im Gewichtsverhältnis von
a) kleiner 2,2 zu 1,0 gemischt werden, um niedrigviskose Knochenzemente mit einer Klebfreiheit nach ISO5833 nach größer 3,0 Minuten nach dem Vermischen herzustellen,
b) von 2,2 bis kleiner 3,3 zu 1,0 gemischt werden, um mittelviskose Knochenzemente mit einer Klebfreiheit nach ISO5833 nach von größer gleich 1,5 bis 3,0 Minuten nach dem Vermischen herzustellen, oder
c) von größer gleich 3,3 zu 1,0 gemischt werden, um hochviskose Knochenzemente mit einer Klebfreiheit nach ISO5833 nach größer gleich 1,0 bis kleiner 1,5 Minuten nach dem Vermischen herzustellen.

5. Verfahren nach einem der Ansprüche 2 bis 4, **dadurch gekennzeichnet, dass**
- eine definierte Menge der Komponente A in Gramm zur Einstellung des Gewichtsverhältnisses a), b) oder c) oder ein dieser Menge entsprechendes Volumen der Komponente A im Innenraum (13) einer Kartusche (1) vorgelegt wird, wobei die Kartusche (1) an ihrem einen Ende einen Kartuschenanschluss aufweist und an ihrem anderen, gegenüberliegenden Ende einen Austragskolben (2) aufweist, wobei durch eine Durchführung in dem Austragskolben eine Mischstange (4) an eine Mischeinrichtung im Inneren (9) der Kartusche anbringbar ist oder angeordnet ist, wobei die Mischeinrichtung von aussen durch Bewegen der Mischstange (4) entlang einer Achse des Innenraumes (13) bedienbar ist, wobei optional der Kartusche eine Verbindungsleitung (14) zugeordnet ist, und,
- die Komponente B in einem Einweg Monomer-Behälter vorgelegt wird.

6. Verfahren nach Anspruch 5, umfassend die Schritte
- Überführen einer definierten Menge der Komponente B in Gramm zur Einstellung des Gewichtsverhältnisses a), b) oder c) oder eines dieser Menge entsprechenden Volumens der Komponente B aus dem Einweg Monomer-Behälter in die Kartusche (1), insbesondere über eine Verbindungsleitung (14), und
- Mischen der Komponente A und B.

7. Verfahren nach Anspruch 6, wobei durch das Mischen der Komponente A und der Komponente B im Gewichtsverhältnis von
a) kleiner 2,2 zu 1,0 niedrigviskose Knochenzemente mit einer Klebfreiheit nach ISO5833 nach größer 3,0 Minuten nach dem Vermischen erhalten werden,
b) von 2,2 bis kleiner 3,3 zu 1,0 mittelviskose Knochenzemente mit einer Klebfreiheit nach ISO5833 nach von größer gleich 1,5 bis 3,0 Minuten nach dem Vermischen erhalten werden, oder
c) von größer gleich 3,3 zu 1,0 hochviskose Knochenzemente mit einer Klebfreiheit nach ISO5833 nach größer gleich 1,0 bis kleiner 1,5 Minuten nach dem Vermischen erhalten werden.

8. Verfahren nach Anspruch 6 oder 7, wobei die Komponente A und die Komponente B im Innenraum (13) mit einer Mischeinrichtung (9) gemischt werden, indem die Mischeinrichtung (9) durch Bewegen einer drehbar und in Längsrichtung verschiebbar in den Innenraum (13) der Kartusche (1) geführten Mischstange (4) bedient wird, wobei bevorzugt nach dem Mischen die Mischstange (4) bis zum Anschlag aus dem Innenraum (13) der Kartusche (1) herausgezogen wird und besonders bevorzugt die Mischstange (4) nach dem Herausziehen bis zum Anschlag an einer Sollbruchstelle abgebrochen wird.

9. Verfahren nach einem der Ansprüche 5 bis 8, wobei die Kartusche (1) mit dem niedrigviskosen, mittelviskosen oder hochviskosen polymerisierbaren Knochenzement von der Verbindungsleitung (14) gelöst wird und der Knochenzement durch Vortreiben eines Austragskolbens (2), der axial beweglich in der Kartusche (1) gelagert ist und der den Innenraum (13) der Kartusche (1) auf einer Seite begrenzt, aus dem Innenraum (13) der Kartusche (1) ausgetragen wird.

## Claims

1. Kit for producing polymerisable bone cements, **characterised in that** it comprises component A and component B, whereby
component A is present as a powder and comprises
(a.1) at least one powder-like polymethylmethacrylate copolymer, whereby the powder-like polymethylmethacrylate copolymer comprises at least one particulate polymethylmethacrylate copolymer with a molar mass Mn of more than / equal to 200,000 g/mol to 1,000,000 g/mol, and the polymethylmethacrylate copolymer can be obtained through polymerisation of a mixture of more than / equal to 90.0% by weight methylmethacrylate and less than / equal to 10.0% by weight to more than / equal to 1% by weight of one or more co-monomers, and the total composition with respect to this mixture corresponds to 100% by weight;
(a.2) at least one powder-like radiopaquer; and
(a.3) at least one polymerisation initiator; and
component B is present as a liquid and comprises
(b.1) at least one monomer for radical polymerisation;
(b.2) optionally, at least one polymerisation accelerator; and
(b.3) optionally, at least one stabiliser; and
whereby component A is present in the internal space (13) of a cartridge (1), whereby the cartridge (1) comprise a cartridge connector with internal thread (8) on its one end and a dispensing plunger (2) on its other, opposite end;
whereby a mixing rod (4) can be attached or is arranged on a mixing facility on the inside (13) of the cartridge through a feedthrough in the dispensing plunger (2), whereby the mixing facility can be operated from outside by moving the mixing rod (4) along an axis of the internal space (13); and;
whereby component B is contained in a monomer container for single or repeated use, whereby component B is present in a glass ampoule with an ampoule head that can be broken off,
whereby the glass ampoule, the cartridge (1) and/or a dosing facility, which is arranged between the monomer container and the cartridge, comprise markers, whereby scale divisions of the markers can be used to read the amount of component B to be added in order to adjust the weight ratio or the volume ratio corresponding to the weight ratio of component A comprising the polymethylmethacrylate copolymer and component B comprising the monomer for radical polymerisation to approximately 2.0 to 3.4 to 1.0.

2. Method for producing a polymerisable bone cement through the use of a kit according to claim 1, by mixing two components A and B with each other, whereby component B is present as a liquid and comprises
(b.1) at least one monomer for radical polymerisation;
(b.2) at least one polymerisation accelerator; and
(b.3) at least one stabiliser;
component A is present as a powder and comprises
(a.1) at least one powder-like polymethylmethacrylate copolymer that is soluble in (b.1) or a powder-like mixture comprising polymethylmethacrylate copolymers that are soluble in (b.1), whereby the polymethylmethacrylate copolymer comprises at least one particulate polymethylmethacrylate copolymer with a molar mass of more than / equal to 200,000 g/mol, and the polymethylmethacrylate copolymer can be obtained through polymerisation of a mixture of more than / equal to 90.0% by weight methylmethacrylate and less than / equal to 10.0% by weight to more than / equal to 1% by weight of one or more co-monomers, and the total composition of the polymethylmethacrylate copolymer with respect to this mixture corresponds to 100% by weight;
(a.2) at least one powder-like radiopaquer; and
(a.3) at least one polymerisation initiator; and
whereby the weight ratio of component A comprising the polymethylmethacrylate copolymer and component B comprising the monomer for radical polymerisation is approximately 2.0 to 3.4 to 1.0; and components A and B are mixed at a weight ratio selected from
a) less than 2.2 to 1.0 or
b) 2.2 to less than 3.3 to 1.0, or
c) more than / equal to 3.3 1.0.

3. Method according to claim 2, **characterised in that** component A comprises from 1.0 to 2.5% by weight of at least one polymerisation initiator, preferably dibenzoyl peroxide, with respect to the total composition of component A being 100% by weight.

4. Method according to claim 3, **characterised in that** component A and component B are mixed at a weight ratio
a) of less than 2.2 to 1.0 in order to produce low-viscosity bone cements with a tack-free time according to ISO5833 of more than 3.0 minutes after the mixing,
b) from 2.2 to less than 3.3 to 1.0 in order to produce medium-viscosity bone cements with a tack-free time according to ISO5833 of more than/equal to 1.5 to 3.0 minutes after the mixing, or
c) of more than / equal to 3.3 to 1.0 in order to produce high-viscosity bone cements with a tack-free time according to ISO5833 of more than / equal to 1.0 to less than 1.5 minutes after the mixing.

5. Method according to any one of the claims 2 to 4, **characterised in that**
- a defined amount of component A, in grams, for adjusting the weight ratio a), b) or c) or a volume of component A corresponding to said amount is provided in the internal space (13) of a cartridge (1), whereby the cartridge (1) comprises a cartridge connector on one of its ends and a dispensing plunger (2) on its other, opposite end, whereby a mixing rod (4) can be attached or is arranged on a mixing facility on the inside (9) of the cartridge through a feedthrough in the dispensing plunger, whereby the mixing facility can be operated from outside by moving the mixing rod (4) along an axis of the internal space (13); whereby, optionally, a connecting line (14) is assigned to the cartridge, and
- component B is provided in a monomer container for single use.

6. Method according to claim 5, comprising the steps of
- transferring a defined amount of component B, in grams, for adjusting the weight ratio a), b) or c) or a volume of component B corresponding to said amount from the monomer container for single use to the cartridge (1) in particular via a connecting line (14), and
- mixing the components A and B.

7. Method according to claim 6, whereby mixing component A and component B at a weight ratio of
a) less than 2.2 to 1.0 allows low-viscosity bone cements with a tack-free time according to ISO5833 of more than 3.0 minutes after the mixing to be obtained,
b) from 2.2 to less than 3.3 to 1.0 allows medium-viscosity bone cements with a tack-free time according to ISO5833 of more than / equal to 1.5 to 3.0 minutes after the mixing to be attained, or
c) more than / equal to 3.3 to 1.0 allows high-viscosity bone cements with a tack-free time according to ISO5833 of more than / equal to 1.0 to less than 1.5 minutes after the mixing to be obtained.

8. Method according to claim 6 or 7, whereby component A and component B are mixed in the internal space (13) by means of a mixing facility (9) through operating the mixing facility (9) through moving a mixing rod (4) that is guided into the internal space (13) of the cartridge (1) such that it can be rotated and shifted in longitudinal direction, whereby, preferably, after the mixing, the mixing rod (4) is pulled out of the internal space (13) of the cartridge (1) to the limit stop, and, particularly preferably, the mixing rod (4) is broken off at a predetermined breakage site after being pulled out to the limit stop.

9. Method according to any one of the claims 5 to 8, whereby the cartridge (1) with the low-viscosity, medium-viscosity or high viscosity polymerisable bone cement is being detached from the connecting line (14) and the bone cement is dispensed from the internal space (13) of the cartridge (1) by propelling a dispensing plunger (2) that is supported in the cartridge (1) such as to be axially mobile and borders the internal space (13) of the cartridge (1) on one side.

## Revendications

1. Kit pour la fabrication de ciments osseux polymérisables, **caractérisé en ce qu'**il comprend le composant A et le composant B,
le composant A étant disponible sous forme de poudre et comprenant
(a.1) au moins un copolymère de polyméthacrylate de méthyle sous forme de poudre, le copolymère de polyméthacrylate de méthyle sous forme de poudre comprenant au moins un copolymère de polyméthacrylate de méthyle particulaire avec une masse molaire Mn supérieure ou égale à une valeur comprise entre 200 000 g/mol et 1 000 000 g/mol et le copolymère de polyméthacrylate de méthyle étant disponible par la polymérisation d'un mélange supérieur ou égal à 90,0 % en poids de méthacrylate de méthyle et inférieur ou égal à 10,0 % en poids à supérieur ou égal à 1 % en poids d'un ou plusieurs comonomères et la composition totale s'élevant à 100 % en poids par rapport à ce mélange,
(a.2) au moins une matière radio-opaque sous forme de poudre et
(a.3) au moins un initiateur de polymérisation ; et
le composant B étant disponible sous forme liquide et comprenant
(b.1) au moins un monomère polymérisable par des radicaux,
(b.2) en option au moins un accélérateur de polymérisation et
(b.3) en option au moins un stabilisateur et
le composant A étant disponible dans l'espace intérieur (13) d'une cartouche (1), la cartouche (1) présentant, à l'une de ses extrémités, un connecteur de cartouche avec filetage intérieur (8) et, à son autre extrémité opposée, un piston d'extraction (2), une tige de mélange (4) pouvant être montée ou étant disposée dans l'espace intérieur (13) de la cartouche sur un dispositif de mélange par une traversée dans le piston d'extraction (2), le dispositif de mélange étant utilisable de l'extérieur par le déplacement de la tige de mélange (4) le long d'un axe de l'espace intérieur (13) et le composant B étant contenu dans un contenant de monomère à usage unique ou réutilisable, le composant B étant disponible dans une ampoule en verre avec tête d'ampoule détachable,
l'ampoule en verre, la cartouche (1) et/ou
un dispositif de dosage qui est disposé entre le contenant de monomère et la cartouche, présentant des marquages, la quantité ajoutée du composant B pour la définition du rapport de poids ou le rapport de volume du composant A, correspondant au rapport de poids, comprenant le copolymère de polyméthacrylate de méthyle par rapport au composant B comprenant le monomère polymérisable par des radicaux, étant lisible sur les marquages en graduations de marquage d'environ 2,0 à 3,4 pour 1,0.

2. Procédé pour la fabrication d'un ciment osseux polymérisable en utilisant un kit conformément à la revendication n°1 par le mélange de deux composants A et B, le composant B étant disponible sous forme de liquide et comprenant
(b.1) au moins un monomère polymérisable par des radicaux,
(b.2) au moins un accélérateur de polymérisation et
(b.3) au moins un stabilisateur,
le composant A étant disponible sous forme de poudre et comprenant
(a.1) au moins un copolymère de polyméthacrylate de méthyle sous forme de poudre soluble dans (b.1) et un mélange sous forme de poudre comprenant des copolymères de polyméthacrylate de méthyle solubles dans (b.1), le copolymère de polyméthacrylate de méthyle comprenant au moins un copolymère de polyméthacrylate de méthyle particulaire avec une masse molaire supérieure ou égale à 200 000 g/mol et le copolymère de polyméthacrylate de méthyle étant disponible par la polymérisation d'un mélange supérieur ou égal à 90,0 % en poids de méthacrylate de méthyle et inférieur ou égal à 10,0 % en poids à supérieur ou égal à 1 % en poids d'un ou plusieurs comonomères et la composition totale du copolymère de polyméthacrylate de méthyle s'élevant à 100 % en poids par rapport à ce mélange,
(a.2) au moins une matière radio-opaque sous forme de poudre et
(a.3) au moins un initiateur de polymérisation ; et
le rapport de poids du composant A comprenant le copolymère de polyméthacrylate de méthyle par rapport au composant B comprenant le monomère polymérisable par des radicaux s'élevant d'environ 2,0 à 3,4 pour 1,0 et
les composants A et B dans le rapport de poids sélectionné
a) inférieur 2,2 pour 1,0 ou
b) de 2,2 à inférieur à 3,3 pour 1,0 ou
c) supérieur ou égal à 3,3 pour 1,0 étant mélangés.

3. Procédé conformément à la revendication n°2, **caractérisé en ce que** le composant A de 1,0 à 2,5 % en poids présente au moins un initiateur de polymérisation, de préférence du peroxyde de dibenzoyle par rapport à la composition totale du composant A de 100% en poids.

4. Procédé conformément à la revendication n°3, **caractérisé en ce que** le composant A et le composant B sont mélangés dans un rapport de poids
a) inférieur à 2,2 pour 1,0 pour fabriquer des ciments osseux à faible viscosité avec une absence de colle suivant ISO5833 après plus de 3,0 minutes après le mélange,
b) de 2,2 à moins de 3,3 pour 1,0 pour fabriquer des ciments osseux à viscosité moyenne avec une absence de colle suivant ISO5833 après 1,5 à 3,0 minutes ou plus après le mélange ou
c) supérieur ou égal à 3,3 pour 1,0 pour fabriquer des ciments osseux à haute viscosité avec une absence de colle suivant ISO5833 après 1,0 à moins de 1,5 minutes ou plus après le mélange.

5. Procédé conformément à l'une des revendications n°2 à n°4, **caractérisé en ce que**
- une quantité définie du composant A en grammes pour la définition du rapport de poids a), b) ou c) ou un volume du composant A correspondant à cette quantité est soumis dans l'espace intérieur (13) d'une cartouche (1), la cartouche (1) présentant, à l'une de ses extrémités, un connecteur de cartouche avec filetage intérieur (8) et, à son autre extrémité opposée, un piston d'extraction (2), une tige de mélange (4) pouvant être montée ou étant disposée à l'intérieur (9) de la cartouche sur un dispositif de mélange par une traversée dans le piston d'extraction (2), le dispositif de mélange étant utilisable de l'extérieur par le déplacement de la tige de mélange (4) le long d'un axe de l'espace intérieur (13), une ligne de liaison (14) étant affectée à la cartouche en option et
- le composant B étant soumis dans un contenant de monomère à usage unique.

6. Procédé conformément à la revendication n°5, comprenant les étapes suivantes :
- transfert d'une quantité définie du composant B en grammes pour la définition du rapport de poids a), b) ou c) ou d'un volume du composant B, correspondant à cette quantité, prélevé du contenant de monomère à usage unique, dans la cartouche (1), en particulier par une ligne de liaison (14) et
- mélange des composants A et B.

7. Procédé conformément à la revendication n°6, par le mélange du composant A et du composant B dans le rapport de poids
a) inférieur à 2,2 pour 1,0, des ciments osseux à faible viscosité avec une absence de colle suivant ISO5833 étant obtenus après plus de 3,0 minutes après le mélange,
b) de 2,2 à moins de 3,3 pour 1,0, des ciments osseux à viscosité moyenne avec une absence de colle suivant ISO5833 étant obtenus après 1,5 à 3,0 minutes ou plus après le mélange ou
c) supérieur ou égal à 3,3 pour 1,0, des ciments osseux à haute viscosité avec une absence de colle suivant ISO5833 étant obtenus après 1,0 à moins de 1,5 minutes ou plus après le mélange.

8. Procédé conformément à la revendication n°6 ou n°7, le composant A et le composant B étant mélangés dans l'espace intérieur (13) avec un dispositif de mélange (9), le dispositif de mélange (9) étant utilisé par le déplacement d'une tige de mélange (4) guidée et déplaçable de façon rotative et dans le sens longitudinal dans l'espace intérieur (13) de la cartouche (1), la cartouche (1) étant retirée de l'espace intérieur (13) de la cartouche (1) jusqu'à la butée de préférence après le mélange et, en particulier de préférence, la tige de mélange (4) étant rompue à un point de rupture prévu après le retrait jusqu'à la butée.

9. Procédé conformément à la revendication n°5 à n°8, la cartouche (1) avec le ciment osseux polymérisable à faible viscosité, moyenne viscosité ou haute viscosité étant détaché de la ligne de liaison (14) et le ciment osseux étant extrait de l'espace intérieur (13) de la cartouche (1) par l'avancement d'un piston d'extraction (2) qui est supporté axialement de façon mobile dans la cartouche (1) et qui limite l'espace intérieur (13) de la cartouche (1) sur un côté.
